(19) Europäisches Patentamt
European Patent Office
Office européen des brevets

(11) Veröffentlichungsnummer: **0 085 899**
**B1**

(12) # EUROPÄISCHE PATENTSCHRIFT

(45) Veröffentlichungstag der Patentschrift: **29.10.86**

(51) Int. Cl.⁴: **C 07 D 471/04**, C 07 D 513/00, A 61 K 31/55 // (C07D471/04, 243:00, 221:00)

(21) Anmeldenummer: **83100730.7**

(22) Anmeldetag: **27.01.83**

(54) **Neue Pyridobenzodiazepinone, Verfahren zu ihrer Herstellung und diese Verbindungen enthaltende Arzneimittel.**

(30) Priorität: **09.02.82 DE 3204403**

(43) Veröffentlichungstag der Anmeldung: **17.08.83 Patentblatt 83/33**

(45) Bekanntmachung des Hinweises auf die Patenterteilung: **29.10.86 Patentblatt 86/44**

(84) Benannte Vertragsstaaten: **AT BE CH DE FR GB IT LI LU NL SE**

(56) Entgegenhaltungen:
**DE - A - 2 724 434**
**DE - A - 2 724 478**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Eberlein, Wolfgang, Dr. Dipl.-Chem., Obere Au 6, D-7950 Biberach 1 (DE)**
Erfinder: **Trummlitz, Günter, Dr. Dipl.-Chem., Buchenweg 27, D-7951 Warthausen (DE)**
Erfinder: **Schmidt, Günther, Dr. Dipl.-Chem., Johann-Seb.-Bach-Strasse 27, D-7950 Biberach 1 (DE)**
Erfinder: **Engel, Wolfhard, Dr. Dipl.-Chem., Mozartstrasse 13, D-7950 Biberach 1 (DE)**
Erfinder: **Hammer, Rudolf, Dr., Via Fabio Filzi, 33, Milano (IT)**
Erfinder: **Del Soldato, Piero, Dr., Via E. Toti 22, Monza (IT)**

**EP 0 085 899 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In den US-Patentschriften 3 660 380, 3 691 159, 4 210 648, 4 213 984 und 4 213 985 werden bereits Pyridobenzodiazepinone mit ulcus-hemmenden und sekretionshemmenden Eigenschaften beschrieben.

Es wurden nun Pyridobenzodiazepinone mit neuartigen Aminoacylresten gefunden, die gegenüber den oben erwähnten Verbindungen interessante und überlegene pharmakologische Eigenschaften aufweisen.

Gegenstand der Erfindung sind somit die neuen Pyridobenzodiazepinone der allgemeinen Formel I

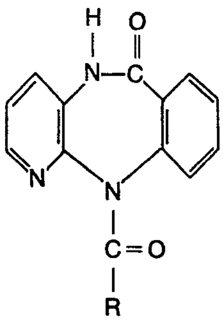

(I)

in der

R einen gegebenenfalls im heterocyclischen Sechsring durch eine oder zwei Methylgruppen substituierten
(1-Methyl-4-piperidin-yl)methyl-,
(1-Methyl-1,2,5,6-tetrahydro-4-pyridinyl)methyl-,
1-Methyl-1,2,5,6-tetrahydro-4-pyridinyl-,
(1-Methyl-4-piperidinyliden)methyl-,
(2,3-Dehydro-8-methyl-8-azabicyclo[3,2,1]-oct-3-yl)-methyl-,
endo- oder exo-(8-Methyl-8-azabicyclo-[3,2,1]oct-3-yl)methyl-Rest
bedeutet, sowie ihre Säureadditionssalze.

Die Verbindungen der allgemeinen Formel I können nach Umsetzung mit physiologisch verträglichen anorganischen oder organischen Säuren auch in Form ihrer physiologisch verträglichen Salze vorliegen. Als Säuren haben sich hierzu beispielsweise Salzsäure, Bromwasserstoffsäure, Schwefelsäure, Methylschwefelsäure, Phosphorsäure, Weinsäure, Fumarsäure, Citronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:

5,11-Dihydro-11-[(1-methyl-4-piperidinyl)acetyl]-6H-pyrido-[2,3-b][1,4]benzodiazepin-6-on,
5,11-Dihydro-11-[(1,3-dimethyl-4-piperidinyl)acetyl]-6H-pyrido-[2,3-b][1,4]benzodiazepin-6-on,
5,11-Dihydro-11-[(1,2-dimethyl-4-piperidinyl)acetyl]-6H-pyrido-[2,3-b][1,4]benzodiazepin-6-on,
cis-5,11-Dihydro-11-[(1,2-dimethyl-4-piperidinyl)acetyl]-6H-pyrido[2,3-b][1,4]benzodiazepin-6-on,
trans-5,11-Dihydro-11-[(1,2-dimethyl-4-piperidinyl)acetyl]-6H-pyrido[2,3-b][1,4]benzodiazepin-6-on,
cis-5,11-Dihydro-11-[(1,3-dimethyl-4-piperidinyl)acetyl]-6H-pyrido[2,3-b][1,4]benzodiazepin-6-on,
trans-5,11-Dihydro-11-[(1,3-dimethyl-4-piperidinyl)acetyl]-6H-pyrido[2,3-b][1,4]benzodiazepin-6-on,
5,11-Dihydro-11-[(1-methyl-1,2,5,6-tetrahydro-4-pyridinyl)-acetyl]-6H-pyrido[2,3-b][1,4]benzodiazepin-6-on,
5,11-Dihydro-11-[(1,2-dimethyl-1,2,5,6-tetrahydro-4-pyridinyl)-acetyl]-6H-pyrido[2,3-b][1,4]benzodiazepin-6-on,
5,11-Dihydro-11-[(1,3-dimethyl-1,2,5,6-tetrahydro-4-pyridinyl)acetyl]-6H-pyrido[2,3-b][1,4]benzodiazepin-6-on,
5,11-Dihydro-11-[(1,5-dimethyl-1,2,5,6-tetrahydro-4-pyridinyl)acetyl]-6H-pyrido[2,3-b][1,4]benzodiazepin-6-on,
5,11-Dihydro-11-[(1,6-dimethyl-1,2,5,6-tetrahydro-4-pyridinyl)acetyl]-6H-pyrido[2,3-b][1,4]benzodiazepin-6-on,
5,11-Dihydro-11-[(1,2,6-trimethyl-1,2,5,6-tetrahydro-4-pyridinyl)carbonyl]-6H-pyrido[2,3-b][1,4]benzodiazepin-6-on,
cis-5,11-Dihydro-4-[(1,2,6-trimethyl-1,2,5,6-tetrahydro-4-pyridinyl)carbonyl]-6H-pyrido[2,3-b][1,4]benzodiazepin-6-on,
trans-5,11-Dihydro-11-[(1,2,6-trimethyl-1,2,5,6-tetrahydro-4-pyridinyl)carbonyl]-6H-pyrido[2,3-b][1,4]benzodiazepin-6-on,
5,11-Dihydro-11-[(1-methyl-1,2,5,6-tetrahydro-4-pyridinyl)-carbonyl]-6H-pyrido[2,3-b][1,4]benzodiazepin-6-on,
5,11-Dihydro-11-[(1,2-dimethyl-1,2,5,6-tetrahydro-4-pyridinyl)-carbonyl]-6H-pyrido[2,3-b][1,4]benzodiazepin-6-on,
5,11-Dihydro-11-[(1,3-dimethyl-1,2,5,6-tetrahydro-4-pyridinyl)carbonyl]-6H-pyrido[2,3-b][1,4]benzodiazepin-6-on,
5,11-Dihydro-11-[(1,5-dimethyl-1,2,5,6-tetrahydro-4-pyridinyl)carbonyl]-6H-pyrido[2,3-b][1,4]benzodiazepin-6-on,
5,11-Dihydro-11-[(1,6-dimethyl-1,2,5,6-tetrahydro-4-pyridinyl)carbonyl]-6H-pyrido[2,3-b][1,4]benzodiazepin-6-on,
endo-5,11-Dihydro-11-[(8-methyl-8-azabicyclo/3,2,1/oct-3-yl)-acetyl]-6H-pyrido[2,3-b][1,4]benzodiazepin-6-on,
exo-5,11-Dihydro-11-[(8-methyl-8-azabicyclo/3,2,1/oct-3-yl)acetyl]-6H-pyrido[2,3-b][1,4]benzodiazepin-6-on,
5,11-Dihydro-11-[(2,3-dehydro-8-methyl-8-azabicyclo[3,2,1]oct-3-yl)acetyl]-6H-pyrido[2,3-b][1,4]benzodiazepin-6-on,
5,11-Dihydro-11-[(1-methyl-4-piperidinyliden)acetyl]-6H-pyrido-[2,3-b][1,4]benzodiazepin-6-on

Ein weiterer Gegenstand der Erfindung sin Arzneimittel, die ein oder mehrere Pyridobenz

diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragees, Kapseln oder Teezubereitungen einarbeiten. Die Tagesdosis liegt im allgemeinen zwischen 0,01 und 5, vorzugsweise 0,02 und 2,5, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die substituierten Pyridobenzodiazepinone der allgemeinen Formel I und ihre Säureadditionssalze besitzen wertvolle Eigenschaften, die sie gewerblich verwertbar machen, insbesondere sind sie durch eine ausgezeichnete Magen- und Darmschutzwirkung bei Warmblütern gekennzeichnet, sie hemmen z.B. die Bildung von Magengeschwüren. Ferner weisen sie, bedingt durch eine geringe Toxizität und das Fehlen wesentlicher Nebenwirkungen, eine günstige therapeutische Breite auf.

Die ausgezeichnete Wirksamkeit der substituierten Pyridobenzodiazepinone der allgemeinen Formel I und ihrer pharmakologisch, d.h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden. Beispielsweise können mit ihnen akute und chronische Ulcera ventriculi und duodeni, Gastritis oder hyperacider Reizmagen bei Mensch oder Tier behandelt werden.

Sollen die erfindungsgemässen substituierten Pyridobenzodiazepinone der allgemeinen Formel I und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Sektretionshemmer, wie $H_2$-Blocker, z.B. Cimetidin, Ranitidin; Magen- und Darmtherapeutika, z.B. Metoclopramid, Bromoprid, Tiaprid; Tranquilizer, wie Benzodiazepine, beispielsweise Diazepam, Oxazepam; Spasmolytika, z.B. Bietamiverin, Camylofin; Anticholinergica, z.B. Oxyphencyclimin, Phencarbamid; Glucocorticoide, wie Prednisolon, Fluocortolon, Betamethason; nicht-steroidale Antiphlogistika, wie Arylessigsäuren und -propionsäuren, Heteroarylessigsäuren und -propionsäuren, Benzothiazincarboxamiddioxide, Pyrazolidindione, Chinazolinone, z.B. Ibuprofen, Naproxen, Diclofenac, Fenbufen, Flurbiprofen, Indometacin, Lonazolac, Sudoxicam, Piroxicam, Phenylbutazon, Bumadizon-Calcium, Proguazon; Lokalanaesthetika, beispielsweise Tetracain, Procain; gegebenenfalls auch Fermente, Vitamine oder Aminosäuren enthalten.

Erfindungsgemäss erhält man die neuen Pyridobenzodiazepinone der allgemeinen Formel I nach folgenden Verfahren:

a) Umsetzung des Pyridobenzodiazepinons der Formel II

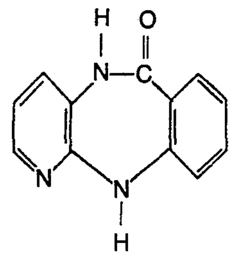

(II)

mit einem Säurederivat der allgemeinen Formel III

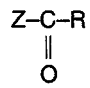

(III)

in der

R die oben angegebene Bedeutung hat und

Z eine nucleofuge Gruppe bzw. Abgangsgruppe darstellt.

Die Umsetzung der Verbindung der allgemeinen Formel II mit den Säurederivaten der allgemeinen Formel III erfolgt in an sich bekannter Weise. Die Abgangsgruppe Z ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte -anhydride, wie sie aus Salzen der entsprechenden Säure (Z=OH) und Säurechloriden, wie Phosphoroxidchlorid, Diphosphorsäuretetrachlorid oder Chlorameisensäureester, gebildet werden oder die bei der Umsetzung von III (Z=OH) mit N-Alkyl-2-halogenpyridiniumsalzen entstehenden N-Alkyl-2-acyloxy-pyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise genannt Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-Dimethylaminopyridin; oder Natriumhydrid. Die Reaktion wird bei Temperaturen zwischen –25 und +130 °C in einem inerten Lösungsmittel durchgeführt. Als inerte Lösungsmittel kommen beispielsweise in Frage chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlor-benzol; polare aprotische Lösungsmittel wie Acetonitril, Dimethylformamid oder Hexamethylphosphorsäuretriamid; oder Gemische davon. Die Reaktionszeiten betragen je nach Menge und Art des eingesetzten Acylierungsmittels der allgemeinen Formel III zwischen 15 Minuten und 80 Stunden. Es ist nicht nötig, die Verbindungen der allgemeinen Formel III in reiner Form herzustellen, sie können vielmehr im Reaktionsansatz in bekannter Weise in situ erzeugt werden.

b) Zur Herstellung von Verbindungen der allgemeinen Formel I, in der R einen gegebenenfalls im heterocyclischen Sechsring durch eine oder zwei Methylgruppen substituierten (1-Methyl-1,2,5,6-tetrahydro-4-pyridinyl)methyl- oder 1-Methyl-1,2,5,6-tetrahydro-4-pyridinyl-Rest bedeutet:

Umsetzung des Pyridobenzodiazepinons der Formel II mit einem Acylierungsmittel der allgemeinen Formel IV

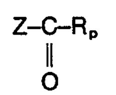

(IV)

in der

Z die oben für die Formel III angegebenen Bedeutungen hat und $R_P$ ein gegebenenfalls methyl- oder dimethylsubstituierter 4-Pyridinyl- oder (4-Pyridinyl)methyl-Rest ist.

Die Acylierung wird gemäss den bei Verfahren a) ausgeführten Bedingungen durchgeführt, die Umsetzung wird jedoch bevorzugt in siedendem Dioxan und in Gegenwart von Pyridin, 4-Dimethylamino-pyridin oder Triethylamin durchgeführt.

Die so erhaltenen Zwischenverbindungen der allgemeinen Formel V

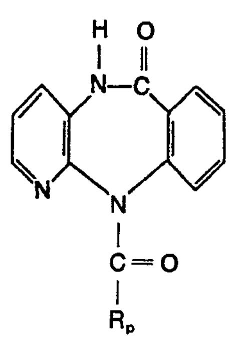

(V)

in der

$R_P$ die oben angegebene Bedeutung hat, werden anschliessend mit Methylierungsmitteln der allgemeinen Formel VI

$$H_3C - X \quad (VI)$$

in der

X den Säurerest von starken Sauerstoffsäuren, beispielsweise von Schwefelsäure, Methylschwefelsäure, Fluorsulfonsäure, Trifluormethansulfonsäure, Methansulfonsäure, Benzolsulfonsäure, p-Toluolsulfonsäure, p-Brombenzolsulfonsäure, Phosphorsäure oder auch Halogenid, bevorzugt Chlorid, Bromid, Jodid bedeutet, zu Pyridiniumsalzen der allgemeinen Formel Va

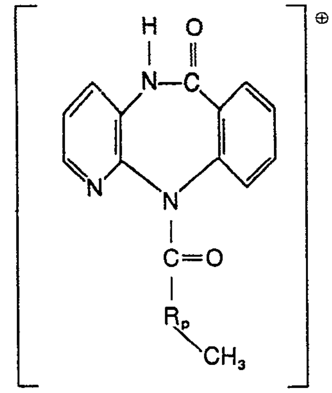

(Va)

in der

$R_P$ und X die vorstehend genannten Bedeutungen haben, methyliert.

Die Methylierung wird in inerten Lösungsmitteln, z.B. chlorierten aliphatischen Kohlenwasserstoffen, wie Methylenchlorid, 1,2-Dichlorethan, offenkettigen oder cyclischen Ethern, wie Diethylether oder Tetrahydrofuran, aromatischen Kohlenwasserstoffen, wie Benzol, Toluol, Xylol oder Dichlorbenzol, bevorzugt jedoch in Dioxan, Acetonitril oder Dimethylformamid und bei Temperaturen zwischen –20 und +130 °C, bevorzugt +30 und 100 °C, durchgeführt.

Anschliessende Reduktion der Pyridiniumsalze Va mit Natrium- oder Kaliumtetrahydridoborat oder Natrium- oder Kaliumalkoxy-, dialkoxy- oder trialkoxyborhydrid in protischen Lösungsmitteln, beispielsweise in Wasser, Methanol, Ethanol, 2-Propanol oder in Gemischen davon bei Temperaturen zwischen – 40 und +50 °C, bevorzugt bei 0 °C, führt zu den gesuchten Pyridobenzodiazepinonen der allgemeinen Formel I, in der R einen (1-Methyl-1,2,5,6-tetrahydro-4-pyridinyl)methyl- oder 1-Methyl-1,2,5,6-tetrahydro-4-pyridinyl-Rest bedeutet.

Die Verfahren zur Herstellung der pharmakologisch wirksamen Pyridobenzodiazepinone der allgemeinen Formel I sind also dadurch gekennzeichnet, dass man das Pyridobenzodiazepinon der Formel II mit Verbindungen der allgemeinen Formel III acyliert oder mit Pyridinalkansäurederivaten der allgemeinen Formel IV umsetzt, anschliessend methyliert und mit Borhydriden oder Alkoxyborhydriden reduziert und jeweils gegebenenfalls anschliessend die erhaltene Base in ein pharmakologisch verträgliches Säureadditionssalz oder ein erhaltenes Säureadditionssalz in die freie Base oder ein pharmakologisch verträgliches Säureadditionssalz überführt.

Ein Teil der erfindungsgemässen Pyridobenzodiazepinone der allgemeinen Formel I enthält ein oder zwei asymmetrische Kohlenstoffatome im Rest R. Diese Verbindungen können deshalb in zwei diastereomeren cis- und trans-Formen oder jeweils als enantiomere (+)- und (–)-Formen auftreten. Die Erfindung umfasst die einzelnen Isomeren ebenso wie ihre Gemische.

Die Trennung der jeweiligen Diastereomeren gelingt auf Grund der unterschiedlichen physikochemischen Eigenschaften, z. B. durch fraktioniertes Umkristallisieren aus geeigneten Lösungsmitteln oder durch chromatographische Verfahren. Jeweils nur ein Diastereomeres wird erhalten, wenn man die oben beschriebene Synthese a) mit nur einem Diastereomeren der allgemeinen Formel III durchführt.

Die Spaltung eventueller Razemate der Verbindungen der allgemeinen Formel I kann nach bekannten Verfahren durchgeführt werden, beispielsweise unter Verwendung einer optisch aktiven Säure, wie (+)- oder (–)-Weinsäure, oder eines Derivats davon, wie (+)- oder (–)-Diacetylweinsäure, (+)- oder (–)-Monomethyltartrat oder (+)-Camphersulfonsäure.

Nach einem üblichen Verfahren zur Isomerentrennung wird das Razemat einer Verbindung der allgemeinen Formel I mit einer der vorstehend angegebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der optisch aktiven Salze in Wasser gelöst, neutralisiert und dadurch die entsprechende freie Verbindung in der (+)- oder (–)-Form erhalten.

Jeweils nur ein Enantiomeres wird auch dadurch erhalten, dass man die oben beschriebene Synthese a) mit nur einem Enantiomeren der allgemeinen Formel III durchführt.

Das als Ausgangsmaterial erforderliche 5,11 Dihydro-6H-pyrido-[2,3-b][1,4]benzodiazepin-6-on der Formel II ist literaturbekannt (siehe US-Patent 3 406 168).

Verbindungen der allgemeinen Formel III und IV sind bekannt oder können in Analogie zu bekannten Herstellungsverfahren leicht erhalten werden. Beispielsweise erhält man bei der Umsetzung von
4-Hydroxy-1-methyl-4-piperidinessigsäure-natriumsalz
mit Thionylchlorid ein Gemisch von
1-Methyl-1,2,5,6-tetrahydro-4-pyridinessigsäurechlorid
und (1-Methyl-4-piperidinyliden)acetylchlorid, das ohne Trennung nach Verfahren a) mit dem Pyridobenzodiazepinon der Formel II zu einem Gemisch der gesuchten Verbindungen der Formel I umgesetzt werden kann, worin R den
(1-Methyl-1,2,5,6-tetrahydro-4-pyridinyl)methyl-
und
(1-Methyl-4-piperidinyliden)methyl-Rest
bedeutet. Dieses Gemisch von Doppelbindungsisomeren kann anschliessend gewünschtenfalls nach üblichen Verfahren, beispielsweise durch fraktionierte Kristallisation, Säulenchromatographie oder Hochdruckflüssigkeitschromatographie, in seine Bestandteile zerlegt werden.

Weiterhin erhält man Tropan-3α-essigsäure und Tropan-3β-essigsäure jeweils frei von dem anderen Isomeren nach W. Schneider u.a., Arch. Pharm. 308, 365–375 (1975) bzw. Ch. L. Zirkle u.a., J. Org. Chem. 27, 1279–1285 (1962). Als reaktive Säurederivate dieser Verbindungen seien die Säurechloride erwähnt, die man in üblicher Weise aus den genannten Carbonsäuren durch Überführung ins Kaliumsalz und anschliessende Behandlung mit gereinigtem Thionylchlorid herstellen kann.

Gegebenenfalls methyl- oder dimethylsubstituierte 4-Pyridinessigsäuren oder Isonicotinsäuren sind käuflich oder können in Analogie zu einer bzw. durch eine Arndt-Eistert-Reaktion über die von D. Jerchel u.a., Liebigs Ann. Chem. 613, 153–170 (1958) bzw. von R. Lukeš u.a., Collect. Czechoslov. Chem. Commun. 23, 1083–1089 (1958); 27, 2220–2222 (1962) beschriebenen substituierten Isonicotinsäuren synthetisiert werden. Als reaktive Säurederivate kommen beispielsweise die Säurechlorid-hydrochloride in Betracht, die nach oder in Analogie zu H. Leditschke, Arch. Pharm. 295, 328 (1962) erhalten werden können.

Wie bereits oben erwähnt, weisen die neuen Verbindungen der allgemeinen Formel I wertvolle pharmakologische Eigenschaften auf; insbesondere besitzen sie antiulcerogene und magensäuresekretionshemmende Wirkungen, sowie günstige Effekte auf verschiedene andere Erkrankungen des Magen-Darm-Traktes, unter denen das Colon irritabile besonders hervorgehoben sei.

Eine günstige Relation zwischen antiulcerogenen und antisekretorischen Wirkungen einerseits und den vor allem bei Therapeutika mit anticholinerger Wirkkomponente auftretenden unerwünschten Wirkungen auf die Pupillenweite und Tränen- und Speichelsekretion andererseits ist für die therapeutische Verwendung der Substanzen von besonderer Wichtigkeit. Die folgenden Versuche zeigen, dass die erfindungsgemässen Verbindungen diesbezüglich überraschend günstige Relationen aufweisen.

Untersuchung auf Selektivität der antimuscarinischen Wirkung

Ziel:

Oxotremorin, ein spezifischer Agonist an muscarinischen Rezeptoren, erzeugt bei Ratten Läsionen der Magenschleimhaut und steigert die Speichelsekretion. Dieses Versuchsmodell wurde gewählt, um eine selektive Wirkung einer antimuscarinischen Substanz auf den Magen erkennen zu können.

Methodik:

Verwendet wurden 10 weibliche Albino-Ratten (Stamm Crl:COBS-CD (SD) BR) pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den Vorversuchen das zu beeinflussende Symptom bei 90 bis 100% der Tiere ausgelöst hatte.

| | |
|---|---|
| Magenschleimhaut-Läsionen: | 0,62 mg/kg i.v. |
| Speichelsekretion: | 0,083 mg/kg i.v. |

Jede antimuscarinische Substanz wurde in gleichmässig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorin-induzierten Speichelsekretion wurde blind durchgeführt, d.h. der Untersucher wusste nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von LITCHFIELD und WILCOXON (J. Pharmacol. Exp. Ther. 96, 99, 1949) ermittelt.

Die Effekte auf Schleimhaut-Läsionen des Magens wurden folgendermassen bewertet:

Die Magenschleimhaut-Läsionen wurden erzeugt durch intravenöse Injektion von 0,62 mg/kg Oxotremorin 30 Minuten nach oraler Gabe von 1 mg/kg Neostigmin (Cholinesterase-Hemmer). 60 Minuten nach Neostigmin-Gabe wurden die Tiere getötet, die Mägen entnommen, geöffnet und auf Vorhandensein von Schleimhaut-Läsionen geprüft. Die schützende Wirkung der Prüfsubstanzen wurde als prozentuale Hemmung (Prozentsatz der Tiere ohne Läsionen) ausgedrückt. $ED_{50}$- bzw. $ED_{70}$-Werte wurden nach der Methode von LITCHFIELD und WILCOXON (s.o.) ermittelt.

Mydriasis

Der Effekt von Testsubstanzen auf die Pupillenweite von Ratten wurde folgendermassen untersucht:

Die Substanzen wurden an Gruppen von jeweils 10 Tieren in wenigstens 3 gleichmässig abgestuften Dosierungen intravenös appliziert. Die Pupillenweite wurde anschliessend 10 Minuten lang auf evtl. Veränderungen (Auftreten von Mydriasis oder Miosis) beobachtet, und zwar wiederum blind, d.h. dem jeweiligen Untersucher war die Vorbehandlung der Tiere unbekannt. Es wurde der Prozentsatz der Versuchstiere ermittelt, bei denen eine Mydriasis auftrat. $ED_{50}$-Werte wurden wiederum nach LITCHFIELD und WILCOXON (s.o.) bestimmt.

2. Bindungsstudien an muscarinischen Rezeptoren:

Bestimmung des $IC_{50}$-Wertes

Als Organspender dienten männliche Sprague-Dawley-Ratten mit 180–220 g Körpergewicht. Nach Entnahme von Herz, Magen und Grosshirnrinde wurden alle weiteren Schritte im eiskalten Hepes-HCl-Puffer (pH 7,4; 100 m molar NaCl, 10 m molar $MgCl_2$) vorgenommen. Die glatte Muskulatur des Magenfundus wurde von der Magenschleimhaut freipräpariert und vorhomogenisiert. Das Gesamtherz wurde mit einer Schere zerkleinert. Alle Organe wurden abschliessend in einem Potter homogenisiert.

Für den Bindungstest wurden die Organhomogenate in folgender Weise verdünnt:

| | |
|---|---|
| Glatter Muskel Magenfundus | 1:100 |
| Gesamtherz | 1:250 |
| Grosshirnrinde | 1:3000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3H$-N-Methylscopolamin ($^3H$-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3H$-NMS. Der Anteil der unspezifischen Bindung wurde definiert als jene Radioaktivität, die in Anwesenheit von 1 µm molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3H$-NMS an die muscarinischen Rezeptoren in den verschiedenen Organen um 50% gehemmt wurde.

Nach den vorstehenden Angaben wurden beispielsweise die folgenden Verbindungen untersucht:

A = 5,11-Dihydro-11-[(1-methyl-1,2,5,6-tetrahydro-4-pyridinyl)-acetyl]-6H-pyrido[2,3-b][1,4]benzodiazepin-6-on

und

B = endo-5,11-Dihydro-11-[(8-methyl-8-azabicyclo[3,2,1]oct-3-yl)acetyl]-6H-pyrido[2,3-b][1,4]benzodiazepin-6-on.

Ergebnisse:

| Substanz | Rezeptor-Bindungs-Test $IC_{50}$ [n Mol $1^{-1}$] Cortex | Glatter Muskel Magenfundus | Herz | Oxotremorin-Test [µg/kg] i.v. Antiulcerogene Wirkung $ED_{50}$ | $ED_{70}$ | Salivationshemmung $ED_{50}$ | Mydriasis $ED_{50}$ [µg/kg] i.v. |
|---|---|---|---|---|---|---|---|
| A | 50 | 200 | 170 | 1,1 | 2 | 28 | 74 |
| B | 50 | 700 | 150 | | 37 | 300 | 220 |

Die Angaben in der vorstehenden Tabelle beweisen, dass die genannten Verbindungen generell eine hohe Affinität zu muscarinischen Rezeptoren besitzen. Darüberhinaus kann man den Daten entnehmen, dass die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus der Grosshirnrinde gegenüber solchen aus der glatten Muskulatur von Magen und Herz.

Aus den pharmakologischen Daten der vorstehenden Tabelle ergibt sich – in völliger Übereinstimmung mit den Rezeptor-Bindungs-Studien-, dass die Entstehung Oxotremorin-induzierter Magenschleimhaut-Läsionen durch die genannten Verbindungen bereits bei Dosierungen gehemmt wird, bei denen noch keine Einschränkung der Speichelsekretion und keine Mydriasis beobachtet wird.

Die folgenden Beispiele dienen zur näheren Erläuterung der Erfindung. «F.» bedeutet «Schmelzpunkt», «Z.» bedeutet «Zersetzung».

Beispiel 1

5,11-Dihydro-11-[(1-methyl-1,2,5,6-tetrahydro-4-pyridinyl)acetyl]-6H-pyrido[2,3-b][1,4]benzodiazepin-6-on

Unter äusserer Kühlung und unter Einhaltung einer Reaktionstemperatur von +15 °C wurde zu einer Suspension von 14,0 g (0,072 Mol) 1-Methyl-1,2,5,6-tetrahydro-4-pyridinessigsäure-Kaliumsalz in 150 ml wasserfreiem Chloroform 9,5 g (0,08 Mol) Thionylchlorid, gelöst in 20 ml Chloroform, zugetropft. Man rührte 20 Minuten nach, engte die Mischung im Vakuum zur Trockne ein und trug den verbleibenden Rückstand in eine Aufschlämmung von 8,4 g (0,04 Mol) 5,11-Dihydro-6H-pyrido [2,3-b][1,4]benzodiazepin-6-on in einem Lösungsmittelgemisch aus 300 ml absolutem Dioxan und 20 ml wasserfreiem Pyridin ein. Unter kräftigem Rühren wurde die Mischung 2 Stunden auf 80 °C erhitzt. Nach dem Erkalten wurde filtriert, der Filterrückstand in Wasser aufgenommen. Man stellte mit festem Natriumcarbonat alkalisch und extrahierte die wässrige Phase erschöpfend mit Chloroform. Die vereinigten Chloroformauszüge wurden getrocknet und im Vakuum vom Lösungsmittel befreit. Der Rückstand wurde an Aluminiumoxid (Aktivitätsstufe I) unter Verwendung von Essigsäureethylester/Methanol (Volumenverhältnis 4:1) zum Eluieren säulenchromatographisch gereinigt. Nach Verreiben mit Essigsäureethylester erhielt man 1,5 g (11% der Theorie) an farblosen Kristallen vom F. 200–202 °C (Z.).

Entsprechend wurden erhalten:

endo-5,11-Dihydro-11-[(8-methyl-8-azabicyclo[3,2,1]oct-3-yl)acetyl]-6H-pyrido[2,3-b][1,4]benzodiazepin-6-on, F. 239–240 °C

exo-5,11-Dihydro-11-[(8-methyl-8-azabicyclo[3,2,1]oct-3-yl)acetyl]-6H-pyrido[2,3,-b][1,4]benzodiazepin-6-on

5,11-Diyhdro-11-[(2,3-dehydro-8-methyl-8-azabicyclo[3,2,1]oct-3-yl)acetyl]-6H-pyrido[2,3-b][1,4]benzodiazepin-6-on, Rf: 0,47 (Methylenchlorid: Cyclohexan: Methanol: Ammoniak wie 68:15:15:2)

Beispiel 2

5,11-Dihydro-11-[(1-methyl-1,2,5,6-tetrahydro-4-pyridinyl)-acetyl]-6H-pyrido[2,3-b][1,4]benzodiazepin-6-on und 5,11-Dihydro-11-[(1-methyl-4-piperidinyliden)acetyl]-6H-pyrido-[2,3-b][1,4]benzodiazepin-6-on

In eine Suspension von 16,8 g (0,08 Mol) des Kaliumsalzes der 4-Hydroxy-1-methyl-4-piperidinessigsäure in 300 ml Chloroform wurde unter äusserer Kühlung und unter Einhaltung einer Reaktionstemperatur von 15 °C eine Lösung von 14,2 g (0,12 Mol) Thionylchlorid in 50 ml Chloroform eingetropft. Nach 20-minütigem Rühren wurde im Vakuum eingedampft, der Rückstand anschliessend in eine Aufschlämmung von 8,4 g (0,04 Mol) 5,11-Dihydro-6H-pyrido-[2,3-b][1,4]benzodiazepin-6-on in einem Gemisch aus 400 ml wasserfreiem Dioxan und 20 ml trockenem Pyridin eingetragen. Unter kräftigem Rühren wurde 2 Stunden unter Rückfluss erhitzt. Nach dem Erkalten wurde der Niederschlag abfiltriert und in Wasser aufgenommen. Die wässrige Lösung wurde mit festem Natriumcarbonat alkalisiert und anschliessend erschöpfend mit Chloroform ausgezogen. Die vereinigten Extrakte wurden getrocknet und im Vakuum eingedampft, der Rückstand anschliessend an Aluminiumoxid (Aktivitätsstufe 1) unter Verwendung von

Essigsäureethylester/Methanol (Volumenverhältnis 99:1) zum Eluieren säulenchromatographiert. Man erhielt 2,5 g (18% der Theorie) an
5,11-Dihydro-11[(1-methyl-1,2,5,6-tetrahydro-4-pyridinyl)acetyl]-6H-pyrido-[2,3-b][1,4]benzodiazepin-6-on,
nach Dünnschichtchromatogramm, IR- und NMR-Spektren völlig identisch mit einem nach Beispiel 1 hergestellten Präparat, sowie 0,3 g (2,2% der Theorie) an
5,11-Dihydro-11-[(1-methyl-4-piperidinyliden)acetyl]-6H-pyrido[2,3-b][1,4]benzodiazepin-6-on.

Beispiel 3

endo-5,11-Dihydro-11-[(8-methyl-8-azabicyclo[3,2,1]oct-3-yl)-acetyl]-6H-pyrido[2,3-b][1,4]benzodiazepin-6-on

Hergestellt analog Beispiel 1 aus Tropan-3α-essigsäure-Kaliumsalz und 5,11-Dihydro-6H-pyrido[2,3-b][1,4]benzodiazepin-6-on in einer Ausbeute von 37% der Theorie.

F. 239–240 °C.

Beispiel 4

5,11-Dihydro-11-[(1-methyl-4-piperidinyl)acetyl]-6H-pyrido-[2,3-b][1,4]benzodiazepin-6-on

Hergestellt analog Beispiel 1 aus 1-Methyl-4-piperidinessigsäure-Kaliumsalz und 5,11-Dihydro-6H-pyrido[2,3-b][1,4]benzodiazepin-6-on in einer Ausbeute von 23% der Theorie. Wachsartige Substanz, F. > 95 °C.

$C_{20}H_{22}N_4O_2$ (350,42)

| | | | |
|---|---|---|---|
| Ber.: | C 68,55 | H 6,33 | N 15,99% |
| Gef.: | C 68,00 | H 6,51 | N 15,37%. |

MS: $\frac{m}{e}$ = 350 (211; 140; 96)

IR ($CH_2Cl_2$): NH 3370/cm; CO 1670/cm, 1680/cm

UV (Ethanol): λ max 300 nm (E=0,14)

UV (Ethanol/KOH): $\lambda_{max}$ 250 nm (E=0,12); (c=50 mg/l; Schichtdicke d=2 mm)

$^1$H-NMR ($CDCl_3/D_2O$; 80 MHz): δ=8,28 (1H-dd; J=5,3 und 1,8 Hz; 2-H); 7,91 Hz (1H-dd; J=7,5 und 1,4 Hz; 4-H) 7,1–7,7 (6H-m; ar.H); 2,5–2,9 (2H-m); 0,8–2,4 (12H-m), davon bei δ=2,18 (3H-s; N-$CH_3$)

Entsprechend erhielt man:

5,11-Diyhdro-11-[(1,3-dimethyl-4-piperidinyl)acetyl]-6H-pyrido-[2,3-b][1,4]benzodiazepin-6-on

5,11-Dihydro-4-[(1,2-dimethyl-4-piperidinyl)acetyl]-6H-pyrido-[2,3-b][1,4]benzodiazepin-6-on

exo-5,11-Dihydro-11-[(8-methyl-8-azabicyclo[3,2,1]oct-3-yl)-acetyl]-6H-pyrido[2,3-b][1,4]benzodiazepin-6-on.

Beispiel 5

5,11-Dihydro-11-[(1-methyl-1,2,5,6-tetrahydro-4-pyridinyl)carbonyl]-6H-pyrido-[2,3-b][1,4]benzodiazepin-6-on

Die Mischung aus 10,6 g (0,05 Mol) 5,11-Dihydro-6H-pyrido [2,3-b][1,4]benzodiazepin-6-on, 10,8 g (0,055 Mol) 1-Methyl-1,2,5,6-tetrahydroisonicotinsäurechlorid-hydrochlorid, 20,7 g (0,15 Mol) Kaliumcarbonat und 200 ml wasserfreiem Toluol wurde unter gutem Rühren 48 Stunden unter Rückfluss gekocht. Nach dem Erkalten wurde in 500 ml Eiswasser eingerührt, die organische Schicht abgetrennt und die wässrige erschöpfend mit Essigsäureethylester extrahiert. Die vereinigten organischen Phasen wurden über Natriumsulfat getrocknet, dann im Vakuum eingedampft. Der Rückstand wurde einmal an Kieselgel 0,1 bis 0,4 mm Korngrösse unter Verwendung von 1,2-Dichlorethan/Methanol (Volumenverhältnis 9:1) und einmal an Kieselgel 0,032 bis 0,063 mm Korngrösse unter Verwendung von Methylenchlorid/Methanol (Volumenverhältnis 10:1) als Eluentien säulenchromatographisch gereinigt. Aufarbeitung der geeigneten Fraktionen in der üblichen Weise ergab farblose Kristalle vom F. 226–227 °C (Diisopropylether/Methanol). Ausbeute: 210 mg (1,3% der Theorie).

Beispiel 6

5,11-Dihydro-11-[(1-methyl-1,2,5,6-tetrahydro-4-pyridinyl)-carbonyl]-6H-pyrido[2,3-b][1,4]benzodiazepin-6-on

a) 5,11-Dihydro-11-[(4-pyridinyl)carbonyl]-6H-pyrido[2,3-b][1,4]benzodiazepin-6-on

Ein Gemisch von 40,4 g (0,191 Mol) 5,11-Diyhdro-6H-pyrido-[2,3-b][1,4]benzodiazepin-6-on, 46,0 g (0,258 Mol) Isonicotinsäurechlorid-hydrochlorid, 42 ml (0,52 Mol) Pyridin und 600 ml wasserfreiem Dioxan wurde 3 Stunden unter Rückfluss gekocht. Der erkaltete Ansatz wurde filtriert der Filterrückstand in 300 ml Wasser aufgenommen und die erhaltene Lösung zweimal mit je 100 ml Dichlormethan ausgeschüttelt. Die wässrige Phase wurde sodaalkalisch gestellt und erschöpfend mit Methylenchlorid extrahiert. Die so erhaltenen Methylenchloridextrakte wurden mit Wasser gewaschen, über Natriumsulfat getrocknet und im Vakuum vom Lösungsmittel befreit. Der Rückstand wurde aus heissem Methanol umkristallisiert. Nach dem Waschen mit Ether schmolzen die beigefarbenen Kristalle bei 265–226 °C.

Ausbeute. 21,0 g (35% der Theorie).

b) 5,11-Dihydro-11-[(4-pyridinyl)carbonyl]-6H-pyrido[2,3-b][1,4]benzodiazepin-6-on-methoiodid

6,3 g (0,02 Mol) 5,11-Dihydro-11-[(4-pyridinyl)carbonyl]-6H-pyrido[2,3-b][1,4]benzodiazepin-6-on wurden in 100 ml wasserfreiem Dimethylformamid gelöst und nach Zugabe von 3,2 g (0,022 Mol) Jodmethan über Nacht bei Zimmertemperatur gerührt. Man engte im Vakuum auf ein Drittel des ursprünglichen Volumens ein, fügte dann ein Gemisch aus gleichen Volumenteilen Methanol und Ether zu, bis das entstandene Salz auszukristallisieren begann. Nach zweistündigem Stehenlassen bei Zimmertemperatur saugte man ab und wusch den Niederschlag gründlich mit Ether

durch. Man erhielt 7,2 g (78% der Theorie) an gelben Kristallen vom F. 290 °C (Z.).

c) 5,11-Dihydro-11-[(1-methyl-1,2,5,6-tetrahydro-4-pyridinyl)-carbonyl]-6H-pyrido[2,3-b][1,4]benzodiazepin-6-on
2,3 g (0,005 Mol)
5,11-Diyhdro-11-[(4-pyridinyl)carbonyl]-6H-pyrido[2,3-b][1,4]benzodiazepin-6-on-methoiodid
wurden in 200 ml Methanol suspendiert und bei 0 °C portionsweise mit 0,25 g (0,006 Mol) Natriumborhydrid versetzt. Es wurde noch 1 Stunde im Eisbad nachgerührt. Dann wurde die Mischung in 1 l Eiswasser eingerührt und mit Methylenchlorid erschöpfend extrahiert. Die vereinigten Extrakte wurden mit Wasser gewaschen, über Natriumsulfat getrocknet und im Vakuum eingeengt. Nach dem Umkristallisieren aus Diisopropylether/Methanol schmolzen die farblosen Kristalle bei 226–227 °C.

Ausbeute: 0,9 g (54% der Theorie).

Das Produkt war nach Dünnschichtchromatogramm, IR- und NMR-Spektrum mit einem nach Beispiel 5 erhaltenen Präparat identisch.

Entsprechend erhielt man:

5,11-Dihydro-11-[(1,2,6-trimethyl-1,2,5,6-tetrahydro-4-pyridinyl)carbonyl]-6H-pyrido-[2,3-b][1,4]benzodiazepin-6-on
5,11-Diyhdro-11-[(1,3-dimethyl-1,2,5,6-tetrahydro-4-pyridinyl)-carbonyl]-6H-pyrido[2,3-b][1,4]benzodiazepin-6-on
und
5,11-Dihydro-11-[(1,5-dimethyl-1,2,5,6-tetrahydro-4-pyridinyl)-carbonyl]-6H-pyrido[2,3-b][1,4]benzodiazepin-6-on
durch Umsetzung von
5,11-Dihydro-6H-pyrido[2,3-b][1,4]benzodiazepin-6-on
mit 3-Methylisonicotinsäurechlorid-hydrochlorid (F. 155–156 °C) über
5,11-Dihydro-11-[(3-methyl-4-pyri-dinyl)carbonyl]-6H-pyrido-[2,3-b][1,4]benzodiazepin-6-on
und
5,11-Dihydro-11-[(3-methyl-4-pyridinyl)carbonyl]-6H-pyrido-[2,3-b][1,4]benzodiazepin-6-on-methoiodid;
aus
5,11-Dihydro-6H-pyrido[2,3-b][1,4]benzodiazepin-6-on
und 2-Methylisonicotinsäurechlorid-hydrochlorid über
5,11-Diyhdro-11-[(2-methyl-4-pyridinyl)-carbonyl]-6H-pyrido-[2,3-b][1,4]benzo-diazepin-6-on
und
5,11-Dihydro-11-[(2-methyl-4-pyridinyl)-carbonyl]-6H-pyrido-[2,3-b][1,4]benzodiazepin-6-on-methoiodid
das
5,11-Dihydro-11-[(1,2-dimethyl-1,2,5,6-tetrahydro-4-pyridinyl)-carbonyl]-6H-pyrido[2,3-b][1,4]benzodiazepin-6-on
und das
5,11-Dihydro-11-[(1,6-dimethyl-1,2,5,6-tetrahydro-4-pyridinyl)-carbonyl]-6H-pyrido[2,3-b][1,4]benzodiazepin-6-on;
aus
5,11-Dihydro-6H-pyrido[2,3-b][1,4]benzodiazepin-6-on
und
4-Pyridinessigsäurechlorid-hydrochlorid über
5,11-Dihydro-11-[(4-pyridinyl)acetyl]-6H-pyrido[2,3-b][1,4]benzodiazepin-6-on
und
5,11-Dihydro-11-[(4-pyridinyl)acetyl]-6H-pyrido[2,3-b][1,4]benzodiazepin-6-on-methoiodid
das
5,11-Dihydro-11-[(1-methyl-1,2,5,6-tetrahydro-4-pyridinyl)-acetyl]-6H-pyrido[2,3-b][1,4]benzodiazepin-6-on.

Beispiel 7

5,11-Dihydro-11-[(1-methyl-1,2,5,6-tetrahydro-4-pyridinyl)-acetyl]-6H-pyrido[2,3-b][1,4]benzodiazepin-6-on

Man erwärmte ein Gemisch von 0,97 g (6,25 Mol) 1-Methyl-1,2,5,6 tetrahydro-4-pyridinessigsäure und 0,20 g (6,25 mMol) 75 proz. Natriumhydrid (in Paraffinöl) in 16 ml Dimethylformamid bei 50–80 °C so lange, bis die Wasserstoffentwicklung beendet war (2 bis 3 Stunden). Zu dem so hergestellten Natriumsalz der Säure fügte man 1,318 g (6,24 mMol)
5,11-Dihydro-6H-pyrido[2,3-b][1,4]benzodiazepin-6-on
und tropfte bei –10 °C 0,99 g 98 proz. Phosphoroxidtrichlorid innerhalb von 10 Minuten zu. Man rührte 4 Stunden bei –10 °C, 4 Stunden bei 0 °C und 20 Stunden bei Zimmertemperatur. Man rührte den Ansatz in 200 g gestossenes Eis ein, stellte mit Natronlauge auf pH 3,5, schüttelte mit Methylenchlorid einmal aus, stellte dann die wässrige Phase auf pH 9 und zog erschöpfend mit Methylenchlorid aus. Man wusch die organische Phase mit Wasser, trocknete sie über Natriumsulfat und engte sie im Vakuum ein. Man erhielt nach säulenchromatographischer Reinigung an Kieselgel (Essigsäureethylester/Methanol im Volumenverhältnis 9:1 zum Eluieren) 0,58 g (27% der Theorie) eines farblosen Produkts, das nach dem Umkristallisieren aus Essigsäureethylester bei 201–202 °C (Z.) schmolz und nach Dünnschichtchromatogramm, Elementaranalyse und IR-Spektrum identisch war mit einem nach Beispiel 1 hergestellten Präparat.

Entsprechend erhielt man:

endo-5,11-Dihydro-11-[(8-methyl-8-azabicyclo[3,2,1]oct-3-yl)-acetyl]-6H-pyrido[2,3-b][1,4]benzodiazepin-6-on,
F. 239–240 °C
exo-5,11-Dihydro-11-[(8-methyl-8-azabicyclo[3,2,1]oct-3-yl)-acetyl]-6H-pyrido[2,3-b][1,4]benzodiazepin-6-on
5,11-Dihydro-11-[(1-methyl-4-piperidinyl)acetyl]-6H-pyrido-[2,3-b][1,4]benzodiazepin-6-on,
nach Dünnschichtchromatogramm und IR- und $^1$H-NMR-Spektrum identisch mit einem nach Beispiel 4 hergestellten Präparat

5,11-Dihydro-11-[(1-methyl-1,2,5,6-tetrahydro-4-pyridinyl)-carbonyl]-6H-pyrido[2,3-b][1,4]benzodiazepin-6-on, F. 226–227 °C (Diisopropylether/Methanol).

Beispiel 8

5,11-Dihydro-11-[(1-methyl-1,2,5,6-tetrahydro-4-pyridinyl)-acetyl]-6H-pyrido[2,3-b][1,4]benzodiazepin-6-on

Zu einer Suspension von 1,552 g (10 mMol) 1-Methyl-1,2,5,6-tetrahydro-4-pyridinessigsäure in 20 ml wasserfreiem Tetrahydrofuran wurden bei 0 °C 1,1 g (10,14 mMol) Chlorkohlensäureethylester zugetropft. Man fügte 2,11 g (10 mMol) 5,11-Dihydro-6H-pyrido[2,3-b][1,4]benzodiazepin-6-on zu dem entstandenen Gemisch, rührte noch 1 Stunde bei 0 °C und anschliessend 4 Stunden bei Zimmertemperatur. Dann rührte man unter äusserer Kühlung mit Eis in 160 ml 2N Natronlauge ein, extrahierte erschöpfend mit Dichlormethan und dampfte die organische Phase im Vakuum zur Trockne ein. Nach säulenchromatographischer Reinigung an Kieselgel unter Verwendung von Essigsäureethylester/Methanol im Volumenverhältnis 9:1 zum Eluieren erhielt man 0,65 g (19% der Theorie) eines farblosen Produkts, das nach Umkristallisieren aus Essigsäureethylester bei 201–202 °C schmolz und nach Dünnschichtchromatogramm, IR- und NMR-Spektrum mit einem nach Beispiel 1 synthetisierten Präparat völlig identisch war.

Analog erhielt man:
aus
5,11-Dihydro-6H-pyrido[2,3-b][1,4]benzodiazepin-6-on
und
1-Methyl-1,2,5,6-tetrahydro-isonicotinsäure
das
5,11-Dihydro-11-[(1-methyl-1,2,5,6-tetrahydro-4-pyridinyl)-carbonyl]-6H-pyrido[2,3-b][1,4]benzodiazepin-6-on
vom F. 226–227 °C (Diisopropylether/Methanol);
aus
5,11-Dihydro-6H-pyrido[2,3-b][1,4]benzodiazepin-6-on
und
Tropan-3α-essigsäure das
endo-5,11-Dihydro-11-[(8-methyl-8-azabicyclo[3,2,1]oct-3-yl)-acetyl]-6H-pyrido[2,3-b][1,4]benzodiazepin-6-on
vom F. 239–240 °C;
aus
5,11-Dihydro-6H-pyrido[2,3-b][1,4]benzodiazepin-6-on
und
1-Methyl-4-piperidinessigsäure das
5,11-Dihydro-11-[(1-methyl-4-piperi-dinyl)acetyl]-6H-pyrido-[2,3-b][1,4]benzo-diazepin-6-on,
nach Dünnschichtchromatogramm, IR- und 1H-NMR-Spektren identisch mit einem nach Beispiel 1 hergestellten Präparat.

Im folgenden wird die Herstellung pharmazeutischer Anwendungsformen anhand einiger Beispiele beschrieben:

Beispiel I

Tabletten mit 5 mg
5,11-Dihydro-11-[(1-methyl-1,2,5,6-tetrahydro-4-pyridinyl)acetyl]-6H-pyrido[2,3-b][1,4]benzodiazepin-6-on

Zusammensetzung:
1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpresst.

| | |
|---|---|
| Tablettengewicht: | 220 mg |
| Stempel: | 9 mm |

Beispiel II

Dragées mit 5 mg
5,11-Dihydro-11-[(1-methyl-1,2,5,6-tetrahydro-4-pyridinyl)-acetyl]-6H-pyrido[2,3-b][1,4]benzododiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 300 mg

Beispiel III

Ampullen mit 1 mg
5,11-Dihydro-11-[(1-methyl-1,2,5,6-tetrahydro-4-pyridinyl)-acetyl]-6H-pyrido[2,3-b][1,4]benzodiazepin-6-on-hydrochlorid

Zusammentsetzung:
1 Ampulle enthält:

| | | |
|---|---|---|
| Wirkstoff | | 1,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschliessend auf das gegebene Volumen aufgefüllt. Die Lösung wird sterilfiltriert und in 1 ml-Ampullen abgefüllt. Sterilisation: 20 Minuten bei 120 °C.

Beispiel IV

Suppositorien mit 5 mg
5,11-Dihydro-11-[(1-methyl-1,2,5,6-tetrahydro-4-pyridinyl)-acetyl]-6H-pyrido[2,3-b][1,4]benzodiazepin-6-on

Zusammentsetzung:
1 Zäpfchen enthält:

| | |
|---|---|
| Wirkstoff | 5,0 mg |
| Zäpfchenmasse (z. B. Witepsol W 45[R]) | 1695,0 mg |
| | 1700,0 mg |

Herstellungsverfahren:
Die feinpulverisierte Wirksubstanz wird in der geschmolzenen und auf 40 °C abgekühlten Zäpfchenmasse suspendiert. Man giesst die Masse bei 37 °C in leicht vorgekühlte Zäpfchenformen aus.
Zäpfchengewicht: 1,7 g

Beispiel V
Tropfen mit
5,11-Dihydro-11-[(1-methyl-1,2,5,6-tetrahydro-4-pyridinyl)-acetyl]-6H-pyrido[2,3-b][1,4]benzodiazepin-6-on

Zusammensetzung:
100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:
Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wässrigen Lösung zu. Abschliessend wird mit Wasser auf 100 ml aufgefüllt und schwebeteilchenfrei filtriert.

**Patentansprüche: für die Vertragsstaaten BE, DE, FR, GB, IT, LU, NL, SE, CH**

1. Pyridobenzodiazepinone der allgemeinen Formel I,

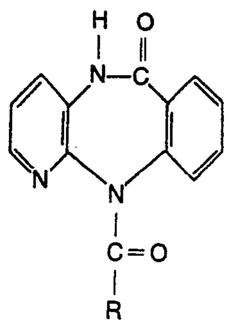

(I)

in der
R eine gegebenenfalls im heterocyclischen Sechsring durch eine oder zwei Methylgruppen substituierte
(1-Methyl-4-piperidinyl)methyl-,
(1-Methyl-1,2,5,6-tetrahydro-4-pyridinyl)methyl-,
1-Methyl-1,2,5,6-tetrahydro-4-pyridinyl-,
(1-Methyl-4-piperidinyliden)methyl-,
2,3-Dehydro-8-methyl-8-azabicyclo[3,2,1]oct-3-yl-methyl-
oder endo- oder
exo-8-Methyl-8-azabicyclo[3,2,1]oct-3-yl-methyl-Gruppe
bedeutet, ihre diastereomeren und enantiomeren Formen und ihre physiologisch verträglichen Säureadditionssalze mit anorganischen oder organischen Säuren.

2. Pyridobenzodiazepinone der allgemeinen Formel I gemäss Anspruch 1, in der
R die (1-Methyl-4-piperidinyl)methyl-, 1-Methyl-1,2,5,6-tetrahydro-4-pyridinyl- oder die endo-8-Methyl-8-azabicyclo[3,2,1] oct-3-yl-methyl-Gruppe bedeutet, und ihre physiologisch verträglichen Säureadditionssalze mit anorganischen oder organischen Säuren.

3. 5,11-Dihydro-11-[(1-methyl-1,2,5,6-tetrahydro-4-pyridinyl)-acetyl]-6H-pyrido[2,3-b][1,4]benzodiazepin-6-on
und dessen physiologisch verträgliche Säureadditionssalze.

4. endo-5,11-Dihydro-11-[(8-methyl-8-azabicyclo[3,2,1]oct-3-yl)-acetyl]-6H-pyrido[2,3-b][1,4]benzodiazepin-6-on
und dessen physiologisch verträgliche Säureadditionssalze.

5. Arzneimittel, enthaltend eine oder mehrere Verbindungen der allgemeinen Formel I gemäss den Ansprüchen 1 bis 4 neben den üblichen Träger- und/oder Hilfsstoffen.

6. Verfahren zur Herstellung neuer Pyridobenzodiazepinone der allgemeinen Formel I

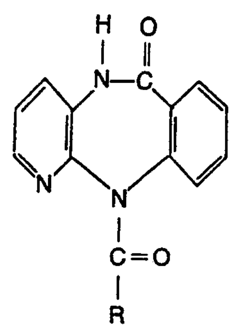

(I)

in der
R eine gegebenenfalls im heterocyclischen Sechsring durch eine oder zwei Methylgruppen substituierte
(1-Methyl-4-piperidinyl)methyl-,
(1-Methyl-1,2,5,6-tetrahydro-4-pyridinyl)methyl-,
1-Methyl-1,2,5,6-tetrahydro-4-pyridinyl-,
(1-Methyl-4-piperidinyliden)methyl-,
2,3-Dehydro-8-methyl-8-azabicyclo[3,2,1]oct-3-yl-methyl-
oder endo oder
exo-8-Methyl-8-azabicyclo[3,2,1]oct-3-yl-methyl-Gruppe
bedeutet, und von deren physiologisch verträglichen Säureadditionssalzen mit anorganischen oder organischen Säuren, dadurch gekennzeichnet, dass

a) das Pyridobenzodiazepinon der Formel II

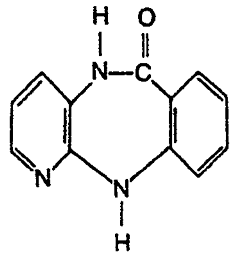

(II)

mit einem Säurederivat der allgemeinen Formel III

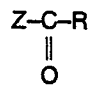

(III)

in der

R wie oben definiert ist und

Z eine nucleofuge Gruppe bedeutet, in einem inerten Lösungsmittel bei Temperaturen zwischen $-25°$ und $+130\,°C$ acyliert wird oder

b) zur Herstellung von Verbindungen der allgemeinen Formel I, in der R eine gegebenenfalls im heterocyclischen Sechsring durch eine oder zwei Methylgruppen substituierte (1-Methyl-1,2,5,6,-tetrahydro-4-pyridinyl)methyl- oder 1-Methyl-1,2,5,6,-tetrahydro-4-pyridinylgruppe bedeutet, ein Pyridiniumsalz der allgemeinen Formel Va

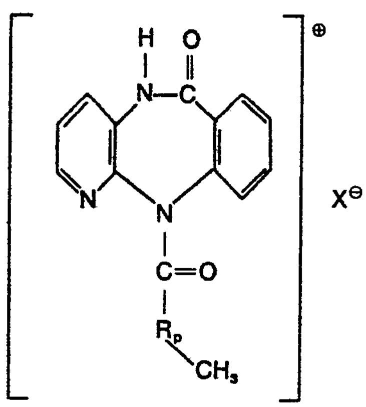

(Va)

in der

X den Säurerest einer starken Sauerstoffsäure oder ein Halogenatom und

$R_P$ eine gegebenenfalls durch eine oder zwei Methylgruppen substituierte 4-Pyridinyl- oder (4-Pyridinyl)methyl-Gruppe bedeuten,

mit einem Borhydrid oder einem Alkoxyborhydrid in protischen Lösungsmitteln bei Temperaturen zwischen $-40$ und $+50\,°C$ reduziert wird,

und gegebenenfalls eine so erhaltene Verbindung der allgemeinen Formel I anschliessend in ihre physiologisch verträglichen Salze mit anorganischen oder organischen Säuren übergeführt wird.

7. Verfahren gemäss Anspruch 6a), dadurch gekennzeichnet, dass als Säurederivat der allgemeinen Formel III ein Säurehalogenid, ein Ester, eine Säureanhydrid, ein gemischtes Säureanhydrid oder ein N-Alkyl-2-acyloxypyridiniumsalz verwendet wird.

8. Verfahren gemäss Anspruch 6a) dadurch gekennzeichnet, dass als Säurederivat der allgemeinen Formel III das gemischte Anhydrid mit einer starken Mineralsäure, verwendet und die Reaktion in einem inerten Lösungsmittel in Gegenwart eines säurebindenden Mittels durchgeführt wird.

9. Verfahren gemäss Anspruch 6b) dadurch gekennzeichnet, dass ein Pyridiniumsalz der allgemeinen Formel Va mittels Natrium- oder Kaliumtetrahydridoborat oder Natrium- oder Kaliumalkoxy-, dialkoxy- oder trialkoxyborhydriden bei $-5$ bis $+10\,°C$ in Anwesenheit eines protischen Lösungsmittels reduziert wird.

10. Verfahren zur Herstellung der Verbindungen der allgemeinen Formel Va gemäss Anspruch 6b), dadurch gekennzeichnet, dass das Pyridobenzodiazepin der Formel II

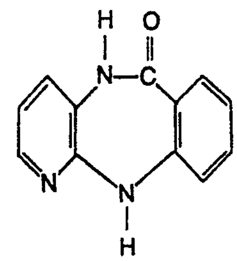

(II)

mit einem Acylierungsmittel der allgemeinen Formel IV

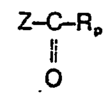

(IV)

in der

Z eine nucleofuge Gruppe, und

$R_P$ eine gegebenenfalls durch ein oder zwei Methylgruppen substituierte 4-Pyridinyl- oder (4-Pyridinyl) methyl-Gruppe darstellen, in einem inerten Lösungsmittel bei Temperaturen bis zum Siedepunkt des Reaktionsgemisches zu einer Verbindung der allgemeinen Formel V

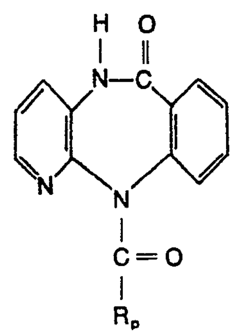

(V)

umgesetzt wird und anschliessend eine so erhaltene Verbindung mit einem Methylierungsmittel der allgemeinen Formel

$$H_3C - X \qquad (VI)$$

in der

X den Säurerest einer starken Sauerstoffsäure oder ein Halogenatom bedeutet, in einem inerten Lösungsmittel bei Temperaturen zwischen $-20$ und $+130\,°C$ methyliert wird.

**Patentansprüche: für den Vertragsstaat AT**

1. Verfahren zur Herstellung neuer Pyridobenzodiazepinone der allgemeinen Formel I

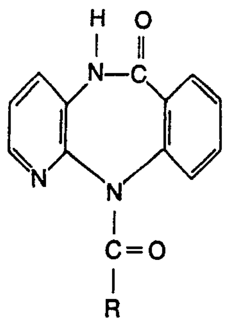

(I)

in der

R eine gegebenenfalls im heterocyclischen Sechsring durch eine oder zwei Methylgruppen substituierte
(1-Methyl-4-piperidinyl)methyl-,
(1-Methyl-1,2,5,6-tetrahydro-4-pyridinyl)methyl-,
1-Methyl-1,2,5,6-tetrahydro-4-pyridinyl-,
(1-Methyl-4-piperidinyliden)methyl-,
2,3-Dehydro-8-methyl-8-azabicyclo[3,2,1]oct-3-yl-methyl-
oder endo oder
exo-8-Methyl-8-azabicyclo[3,2,1]oct-3-yl-methyl-Gruppe
bedeutet, und von deren physiologisch verträglichen Säureadditionssalzen mit anorganischen oder organischen Säuren, dadurch gekennzeichnet, dass

a) das Pyridobenzodiazepinon der Formel II

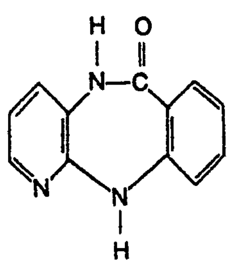

(II)

mit einem Säurederivat der allgemeinen Formel III

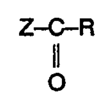

(III)

in der

R wie oben definiert ist und

Z eine nucleofuge Gruppe bedeutet, in einem inerten Lösungsmittel bei Temperaturen zwischen −25 und +130 °C acyliert wird oder

b) zur Herstellung von Verbindungen der allgemeinen Formel I, in der R eine gegebenenfalls im heterocyclischen Sechsring durch eine oder zwei Methylgruppen substituierte
(1-Methyl-1,2,5,6-tetrahydro-4-pyridinyl)methyl-
oder
1-Methyl-1,2,5,6-tetrahydro-4-pyridinylgruppe
bedeutet, ein Pyridiniumsalz der allgemeinen Formel Va

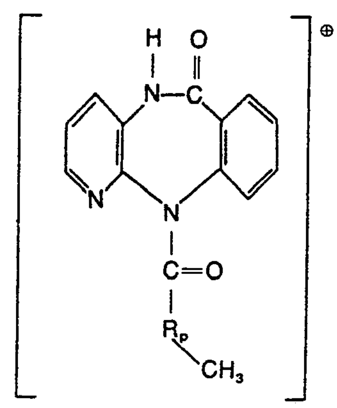

(Va)

in der

X den Säurerest einer starken Sauerstoffsäure oder ein Halogenatom und

$R_P$ eine gegebenenfalls durch eine oder zwei Methylgruppen substituierte 4-Pyridinyl- oder (4-Pyridinyl)methyl-Gruppe bedeuten,

mit einem Borhydrid oder einem Alkoxyborhydrid in protischen Lösungsmitteln bei Temperaturen zwischen −40 und +50 °C reduziert wird,

und gegebenenfalls eine so erhaltene Verbindung der allgemeinen Formel I anschliessend in ihre physiologisch verträglichen Salze mit anorganischen oder organischen Säuren übergeführt wird.

2. Verfahren zur Herstellung neuer Pyridobenzodiazepinone der allgemeinen Formel I

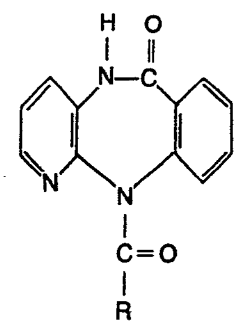

(I)

in der

R eine gegebenenfalls im heterocyclischen Sechsring durch eine oder zwei Methylgruppen substituierte (1-Methyl-4-piperidinyl) methyl-, (1-Methyl-1,2,5,6-tetrahydro-4-pyridinyl)methyl-, 1-Methyl-1,2,5,6-tetrahydro-4-pyridinyl-, (1-Methyl-4-piperidinyliden)methyl-, endo oder exo-8-Methyl-8-azabicyclo[3,2,1] oct-3-yl-methyl-Gruppe bedeutet, und von deren physiologisch verträglichen Säureadditionssalzen mit anorganischen oder organischen Säuren, dadurch gekennzeichnet, dass

a) das Pyridobenzodiazepinon der Formel II

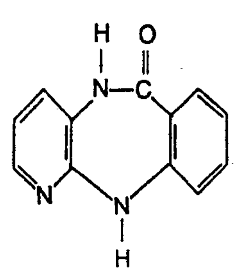

(II)

mit einem Säurederivat der allgemeinen Formel III

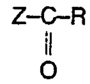
(III)

in der
R wie oben definiert ist und
Z eine nucleofuge Gruppe bedeutet, in einem inerten Lösungsmittel bei Temperaturen zwischen −25 und +130 °C acyliert wird oder

b) zur Herstellung von Verbindungen der allgemeinen Formel I, in der R eine gegebenenfalls im heterocyclischen Sechsring durch eine oder zwei Methylgruppen substituierte (1-Methyl-1,2,5,6-tetrahydro-4-pyridinyl)methyl- oder 1-Methyl-1,2,5,6-tetrahydro-4-pyridinylgruppe bedeutet, ein Pyridiniumsalz der allgemeinen Formel Va

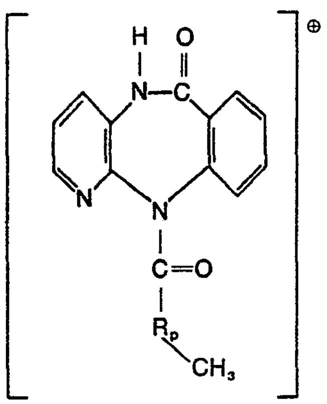
(Va)

in der
X den Säurerest einer starken Sauerstoffsäure oder ein Halogenatom und
$R_p$ eine gegebenenfalls durch eine oder zwei Methylgruppen substituierte 4-Pyridinyl- oder (4-Pyridinyl)methyl-Gruppe bedeuten,
mit einem Borhydrid oder einem Alkoxyborhydrid in protischen Lösungsmitteln bei Temperaturen zwischen −40 und +50 °C reduziert wird,
und gegebenenfalls eine so erhaltene Verbindung der allgemeinen Formel I anschliessend in ihre physiologisch verträglichen Salze mit anorganischen oder organischen Säuren übergeführt wird.

3. Verfahren gemäss Anspruch 1a), dadurch gekennzeichnet, dass als Säurederivat der allgemeinen Formel III ein Säurehalogenid, ein Ester, ein Säureanhydrid, ein gemischtes Säureanhydrid oder ein N-Alkyl-2-acyloxypyridiniumsalz verwendet wird.

4. Verfahren gemäss Anspruch 1a), dadurch gekennzeichnet, dass als Säurederivat der allgemeinen Formel III das gemischte Anhydrid mit einer starken Mineralsäure verwendet und die Reaktion in einem inerten Lösungsmittel in Gegenwart eines säurebindenden Mittels durchgeführt wird.

5. Verfahren gemäss Anspruch 1b), dadurch gekennzeichnet, dass ein Pyridiniumsalz der allgemeinen Formel Va mittels Natrium- oder Kaliumtetrahydridoborat oder Natrium- oder Kaliumalkoxy-, dialkoxy- oder trialkoxyborhydriden bei −5 bis +10 °C in Anwesenheit eines protischen Lösungsmittels reduziert wird.

6. Verfahren zur Herstellung der Verbindungen der allgemeinen Formel Va gemäss Anspruch 1b), dadurch gekennzeichnet, dass das Pyridobenzodiazepinon der Formel II

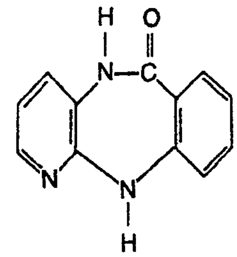
(II)

mit einem Acylierungsmittel der allgemeinen Formel IV

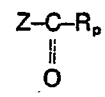
(IV)

in der
Z eine nucleofuge Gruppe und
$R_p$ eine gegebenenfalls durch ein oder zwei Methylgruppen substituierte 4-Pyridinyl- oder (4-Pyridinyl)methyl-Gruppe darstellen, in einem inerten Lösungsmittel bei Temperaturen bis zum Siedepunkt des Reaktionsgemisches zu einer Verbindung der allgemeinen Formel V

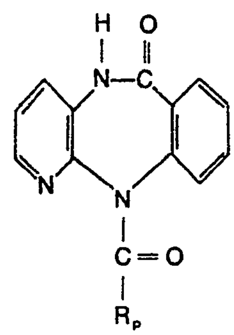
(V)

umgesetzt wird und anschliessend eine so erhaltene Verbindung mit einem Methylierungsmittel der allgemeinen Formel

$$H_3C-X \qquad (VI)$$

in der
X den Säurerest einer starken Sauerstoffsäure oder ein Halogenatom bedeutet, in einem inerten Lösungsmittel bei Temperaturen zwischen −20 und +130 °C methyliert wird.

**Claims for the contracting states: BE, DE, FR, GB, IT, LU, NL, SE, CH, LI**

1. Pyridobenzodiazepinones of general formula

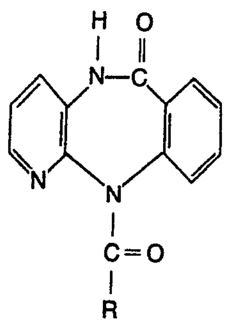

(I)

wherein
R represents a
(1-methyl-4-piperidinyl)methyl,
(1-methyl-1,2,5,6-tetrahydro-4-pyridinyl)methyl,
1-methyl-1,2,5,6-tetrahydro-4-pyridinyl,
(1-methyl-4-piperidinylidene)methyl,
2,3-dehydro-8-methyl-8-azabicyclo[3,2,1]oct-3-yl-methyl,
endo- or
exo-8-methyl-8-azabicyclo[3,2,1]oct-3-yl-methyl
group, each group being optionally substituted in the heterocyclic six-membered ring by one or two methyl groups, the diastereomeric and enantiomeric forms thereof and the physiologically acceptable acid addition salts thereof with inorganic or organic acids.

2. Pyridobenzodiazepinones of general formula I as claimed in claim 1, wherein
R represents the
(1-methyl-4-piperidinyl)methyl,
1-methyl-1,2,5,6-tetrahydro-4-pyridinyl
or
endo-8-methyl-8-azabicyclo[3,2,1]oct-3-yl-methyl
group, and the physiologically acceptable acid addition salts thereof with inorganic or organic acids.

3. 5,11-Dihydro-11-[(1-methyl-1,2,5,6-tetrahydro-4-pyridinyl)-acetyl]-6H-pyrido[2,3-b][1,4]benzodiazepin-6-one
and the physiologically acceptable acid addition salts thereof.

4. endo-5,11-Dihydro-11-[(8-methyl-8-azabicyclo[3,2,1]-oct-3-yl)-acetyl]-6H-pyrido[2,3-b][1,4]benzodiazepin-6-one
and the physiologically acceptable salts thereof.

5. Pharmaceutical compositions containing one or more compounds of general formula I as claimed in claims 1 to 4 together with the usual carriers and/or excipients.

6. Process for preparing new pyridobenzodiazepinones of general formula I

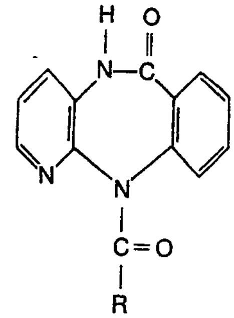

(I)

wherein
R represents a
(1-methyl-4-piperidinyl)methyl,
(1-methyl-1,2,5,6-tetrahydro-4-pyridinyl)methyl,
1-methyl-1,2,5,6-tetrahydro-4-pyridinyl,
(1-methyl-4-piperidinylidene)methyl,
2,3-dehydro-8-methyl-8-azabicyclo[3,2,1]oct-3-yl-methyl,
endo- or
exo-8-methyl-8-azabicyclo[3,2,1]oct-3-yl-methyl
group, each group being optionally substituted in the heterocyclic six-membered ring by one or two methyl groups, and of the physiologically acceptable acid addition salts thereof with inorganic or organic acids, characterised in that

a) the pyridobenzodiazepinone of formula II

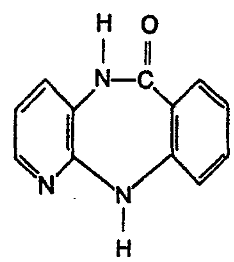

(II)

is acylated with an acid derivative of general formula III

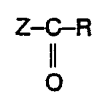

(III)

wherein R is as hereinbefore defined and Z represents a nucleophobic group, in an inert solvent at temperatures between −25 °C and +130 °C, or

b) in order to prepare compounds of general formula I wherein R represents a
(1-methyl-1,2,5,6-tetrahydro-4-pyridinyl)methyl
or 1-methyl-1,2,5,6-tetrahydro-4-pyridinyl group optionally substituted by one or two methyl groups in the heterocyclic ring, a pyridinium salt of general formula

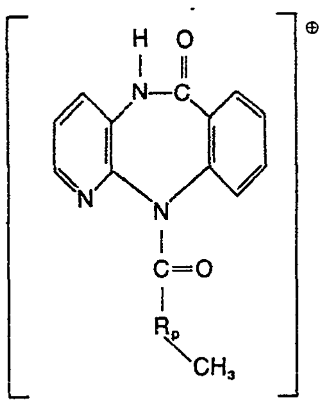

(Va)

wherein

X represents the acid group of a strong oxyacid or a halogen atom and

$R_P$ represents a 4-pyridinyl or (4-pyridinyl)-methyl group optionally substituted by one or two methyl groups,

is reduced with a borohydride or an alkoxyborohydride in protic solvents at temperatures of between −40 °C and +50 °C,

and if desired a compound of general formula I thus obtained is subsequently converted into the physiologically acceptable salts thereof with inorganic or organic acids.

7. Process as claimed in claim 6a, characterised in that an acid halide, an ester, an acid anhydride, a mixed acid anhydride or an N-alkyl-2-acyloxypyridinium salt is used as the acid derivative of general formula III.

8. Process as claimed in claim 6a, characterised in that the mixed anhydride with a strong mineral acid is used as the acid derivative of general formula III and the reaction is effected in an inert solvent in the presence of an acid-binding agent.

9. Process as claimed in claim 6b, characterised in that a pyridinium salt of general formula Va is reduced by means of sodium or potassium tetrahydridoborate or sodium or potassium alkoxy-, dialkoxy- or trialkoxyborohydrides at −5 to +10 °C in the presence of a protic solvent.

10. Process for the preparation of compounds of general formula Va as claimed in claim 6b, characterised in that a pyridobenzodiazepinone of general formula

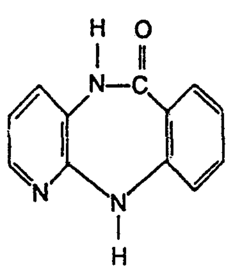

(II)

is reacted with an acylating agent of general formula

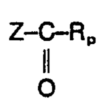

(IV)

wherein Z represents a nucleophobic group, and

$R_P$ represents a 4-pyridinyl or (4-pyridinyl)methyl group otpionally substituted by one or two methyl groups, in an inert solvent at temperatures up to the boiling point of the reaction mixture, to form a compound of general formula

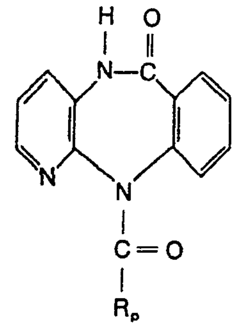

(V)

and subsequently a compound thus obtained is methylated with a methylating agent of general formula

$$H_3C-X \qquad (VI)$$

wherein X represents the acid group of a strong oxyacid or a halogen atom, in an inert solvent at temperatures of between −20 °C and +130 °C.

**Claims for the contracting state: AT**

1. Process for preparing new pyridobenzodiazepinones of general formula I

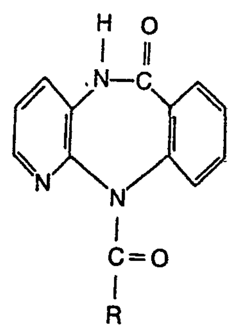

(I)

wherein

R represents a
(1-methyl-4-piperidinyl)methyl,
(1-methyl-1,2,5,6-tetrahydro-4-pyridinyl)methyl,
1-methyl-1,2,5,6-tetrahydro-4-pyridinyl,
(1-methyl-4-piperidinylidene)methyl,
2,3-dehydro-8-methyl-8-azabicyclo[3,2,1]oct-3-yl-methyl,
endo- or
exo-8-methyl-8-azabicyclo[3,2,1]-oct-3-yl-methyl
group, each group being optionally substituted in the heterocyclic six-membered ring by one or two methyl groups, and of the physiologically acceptable acid addition salts thereof with inorganic or organic acids, characterised in that

a) the pyridobenzodiazepinone of formula II

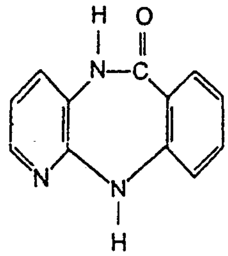

(II)

is acylated with an acid derivative of general formula III

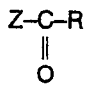

(III)

wherein R is as hereinbefore defined and Z represents a nucleophobic group, in an inert solvent at temperatures between −25 °C and +130 °C, or

b) in order to prepare compounds of general formula I wherein R represents a (1-methyl-1,2,5,6-tetrahydro-4-pyridinyl)methyl or 1-methyl-1,2,5,6-tetrahydro-4-pyridinyl group optionally substituted by one or two methyl groups in the heterocyclic ring, a pyridinium salt of general formula

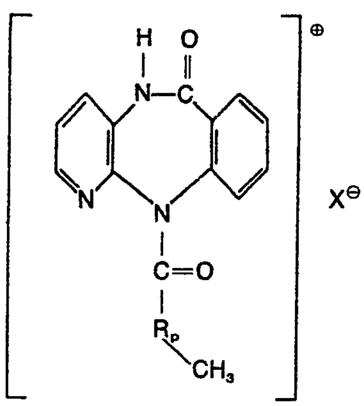

(Va)

wherein
X represents the acid group of a strong oxyacid or a halogen atom and
$R_P$ represents a 4-pyridinyl or (4-pyridinyl)-methyl group optionally substituted by one or two methyl groups,
is reduced with a borohydride or an alkoxyborohydride in protic solvents at temperatures of between −40 °C and +50 °C,
and if desired a compound of general formula I thus obtained is subsequently converted into the physiologically acceptable salts thereof with inorganic or organic acids.

2. Process for preparing new pyridobenzodiazepinones of general formula I

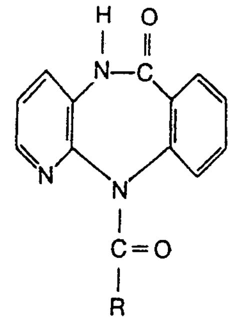

(I)

wherein
R represents a
(1-methyl-4-piperidinyl)methyl,
(1-methyl-1,2,5,6-tetrahydro-4-pyridinyl)methyl,
1-methyl-1,2,5,6-tetrahydro-4-pyridinyl,
(1-methyl-4-piperidinylidene)methyl,
endo- or
exo-8-methyl-8-azabicyclo[3,2,1]oct-3-yl-methyl group, each group being optionally substituted in the heterocyclic sixmembered ring by one or two methyl groups, and of the physiologically acceptable acid addition salts thereof with inorganic or organic acids, characterised in that
a) the pyridobenzodiazepinone of formula II

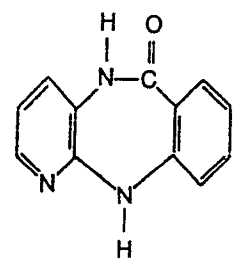

(II)

is acylated with an acid derivative of general formula III

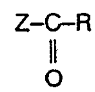

(III)

wherein R is as hereinbefore defined and Z represents a nucleophobic group, in an inert solvent at temperatures between −25 °C and +130 °C, or
b) in order to prepare compounds of general formula I wherein R represents a
(1-methyl-1,2,5,6-tetrahydro-4-pyridinyl)methyl
or
1-methyl-1,2,5,6-tetrahydro-4-pyridinyl
group optionally substituted by one or two methyl groups in the heterocyclic ring, a pyridinium salt of general formula

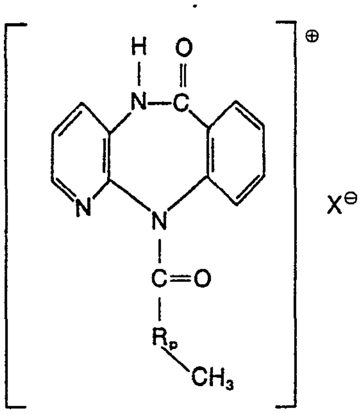

(Va)

wherein

X represents the acid group of a strong oxyacid or a halogen atom and

$R_p$ represents a 4-pyridinyl or (4-pyridinyl)-methyl group optionally substituted by one or two methyl groups,

is reduced with a borohydride or an alkoxyborohydride in protic solvents at temperatures of between −40 °C and +50 °C,

and if desired a compound of general formula I thus obtained is subsequently converted into the physiologically acceptable salts thereof with inorganic or organic acids.

3. Process as claimed in claim 1a, characterised in that an acid halide, an ester, an acid anhydride, a mixed acid anhydride or an N-alkyl-2-acyloxypyridinium salt is used as the acid derivative of general formula III.

4. Process as claimed in claim 1a, characterised in that the mixed anhydride with a strong mineral acid is used as the acid derivative of general formula III and the reaction is effected in an inert solvent in the presence of an acid-binding agent.

5. Process as claimed in claim 1b, characterised in that a pyridinium salt of general formula Va is reduced by means of sodium or potassium tetrahydridoborate or sodium or potassium alkoxy-, dialkoxy- or trialkoxyborohydrides at −5 to +10 °C in the presence of a protic solvent.

6. Process for the preparation of compounds of general formula Va as claimed in claim 1b, characterised in that a pyridobenzodiazepinone of general formula

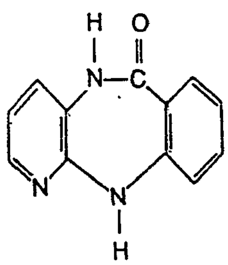

(II)

is reacted with an acylating agent of general formula

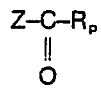

(IV)

wherein Z represents a nucleophobic group, anc

$R_p$ represents a 4-pyridinyl or (4-pyridinyl)methyl group optionally substituted by one or two methyl groups, in an inert solvent at temperatures up to the boiling point of the reaction mixture, to forn a compound of general formula

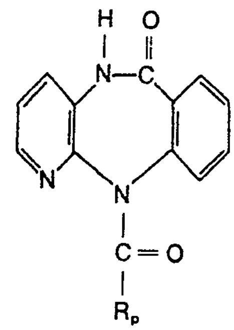

(V

and subsequently a compound thus obtained is methylated with a methylating agent of genera formula

$$H_3C - X \quad \text{(VI)}$$

wherein X represents the acid group of a stronς oxyacid or a halogen atom, in an inert solvent a temperatures of between −20 °C and +130 °C.

**Revendications pour les états contractants: BE DE, FR, GB, IT, LU, NL, SE, CH, LI**

1. Pyridobenzodiazépinones de formule générale I:

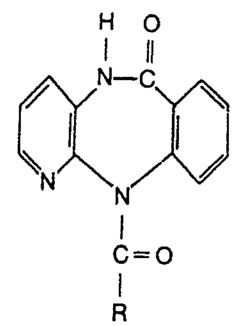

(I

dans laquelle

R représente un groupe
(1-méthyl-4-pipéridinyl)méthyle,
(1-méthyl-1,2,5,6-tétrahydro-4-pyridinyl)méthyle,
1-méthyl-1,2,5,6-tétrahydro-4-pyridinyle,
(1-méthyl-4-pipéridinylidène)méthyle,
2,3-déhydro-8-méthyl-8-azabicyclo[3,2,1]oct-3-yl-méthyle
ou endo- ou
exo-8-méthyl-8-azabicyclo[3,2,1]oct-3-yl-méthyle,
éventuellement substitué dans le cycle hétérocyclique à 6 chaînons par un ou deux groupes méthyle, leurs formes diastéréoisomères et énantiomères et leurs sels d'addition d'acides physiologiquement supportables avec des acides minérau› ou organiques.

2. Pyridobenzodiazépinones de formule générale I selon la revendication 1, dans laquelle
R représente le groupe
(1-méthyl-4-pipéridinyl)méthyle,
1-méthyl-1,2,5,6-tétrahydro-4-pyridinyle
ou le groupe
endo-8-méthyl-8-azabicyclo[3,2,1]oct-3-yl-méthyle,
et leurs sels d'addition d'acides physiologiquement supportables avec des acides minéraux ou organiques.

3. La 5,11-dihydro-11-[(1-méthyl-1,2,5,6-tétrahydro-4-pyridinyl)acétyl]-6H-pyrido[2,3-b][1,4]benzodiazépine-6-one
et ses sels d'addition d'acides physiologiquement supportables.

4. L'endo-5,11-dihydro-11-[(8-méthyl-8-azabicyclo[3,2,1]oct-3-yl)acétyl]-6H-pyrido[2,3-b][1,4]benzodiazépine-6-one
et ses sels d'addition d'acides physiologiquement supportables.

5. Médicaments contenant un ou plusieurs composés de formule générale I selon les revendications 1 à 4, conjointement aux excipients et/ou adjuvants usuels.

6. Procédé pour la préparation de nouvelles pyridobenzodiazépinones de formule générale I

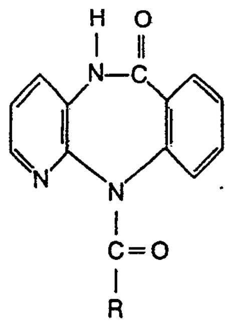

(I)

dans laquelle
R représente un groupe
(1-méthyl-4-pipéridinyl)méthyle,
(1-méthyl-1,2,5,6-tétrahydro-4-pyridinyl)méthyle,
1-méthyl-1,2,5,6-tétrahydro-4-pyridinyle,
(1-méthyl-4-pipéridinylidène)méthyle,
2,3-déhydro-8-méthyl-8-azabicyclo[3,2,1]oct-3-yl-méthyle
ou endo- ou
exo-8-méthyl-8-azabicyclo[3,2,1]oct-3-yl-méthyle,
éventuellement substitué dans le cycle hétérocyclique à 6 chaînons par un ou deux groupes méthyle, et de leurs sels d'addition d'acides physiologiquement supportables avec des acides minéraux ou organiques, caractérisé en ce que

a) on acyle la pyridobenzodiazépinone de formule II

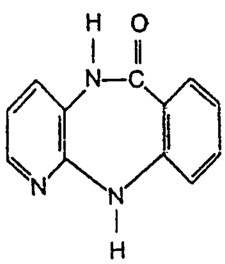

(II)

au moyen d'un dérivé d'acide de formule générale III

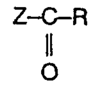

(III)

dans laquelle
R est défini comme plus haut, et
Z représente un groupe nucléofuge, dans un solvant inerte à des températures entre −25 et +130 °C ou

b) pour la préparation de composés de formule générale I dans laquelle R représente un groupe
(1-méthyl-1,2,5,6-tétrahydro-4-pyridinyl)méthyle
ou
1-méthyl-1,2,5,6-tétrahydro-4-pyridinyle,
éventuellement substitué dans le cycle hétérocyclique à 6 chaînons par un ou deux groupes méthyle, on réduit un sel de pyridinium de formule générale Va

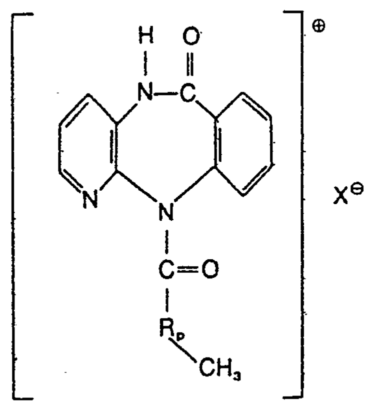

(Va)

dans laquelle
X représente le radical acide d'un acide oxygéné fort ou un atome d'halogène et
$R_P$ représente un groupe 4-pyridinyle ou (4-pyridinyl) méthyle, éventuellement substitué par un ou deux groupes méthyle, au moyen d'un borohydrure ou d'un alcoxyborohydrure dans des solvants protiques, à des températures entre −40 et +50 °C,
et éventuellement, on transforme ensuite un composé ainsi obtenu de formule générale I en ses sels physiologiquement supportables avec des acides minéraux ou organiques.

7. Procédé selon la revendication 6a), caractérisé en ce que l'on utilise, en tant que dérivé d'acide de formule générale III, un halogénure d'acide, un ester, un anhydride d'acide, un anhydride mixte d'acide ou un sel de N-alcoyl-2-acyloxypyridinium.

8. Procédé selon la revendication 6a), caractérisé en ce que l'on utilise, en tant que dérivé d'acide de formule générale III, l'anhydride mixte avec un acide minéral fort et en ce qu'on effectue la réaction dans un solvant inerte en présence d'un agent fixant les acides.

9. Procédé selon la revendication 6b), caractérisé en ce qu'on réduit un sel de pyridinium de formule générale Va au moyen de tétrahydroborate de sodium ou de potassium ou d'alcoxy-,

dialcoxy- ou trialcoxyborohydrures de sodium ou de potassium à $-5$ jusqu'à $+10\,°C$ en présence d'un solvant protique.

10. Procédé pour la préparation des composés de formule générale Va selon la revendication 6b), caractérisé en ce qu'on fait réagir la pyridobenzodiazépinone de formule II

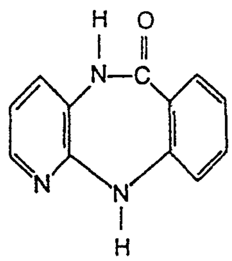

(II)

avec un agent d'acylation de formule générale IV

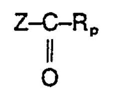

(IV)

dans laquelle

Z représente un groupe nucléofuge, et

$R_p$ représente un groupe 4-pyridinyle ou (4-pyridinyl) méthyle, éventuellement substitué par un ou deux groupes méthyle, dans un solvant inerte à des températures allant jusqu'à la température d'ébullition du mélange réactionnel, pour donner un composé de formule générale V

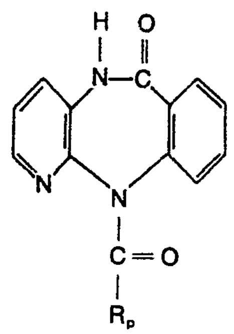

(V)

et en ce qu'ensuite on méthyle un composé ainsi obtenu au moyen d'un agent de méthylation de formule générale

$$H_3C - X \qquad (VI)$$

dans laquelle

X représente le radical acide d'un acide oxygéné fort ou atome d'halogène, dans un solvant inerte à des températures entre $-20$ et $+130\,°C$.

**Revendications pour l'état contractant: AT**

1. Procédé pour la préparation de nouvelles pyridobenzodiazépinones de formule générale I

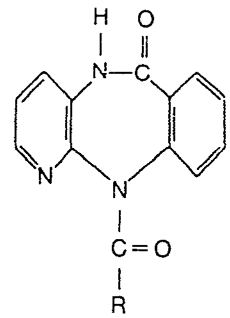

(I

dans laquelle

R représente un groupe (1-méthyl-4-pipéridinyl)méthyle, (1-méthyl-1,2,5,6-tétrahydro-4-pyridinyl)méthyle, 1-méthyl-1,2,5,6-tétrahydro-4-pyridinyle, (1-méthyl-4-pipéridinylidène)méthyle, 2,3-déhydro-8-méthyl-8-azabicyclo[3,2,1]oct-3-yl-méthyle ou endo- ou exo-8-méthyl-8-azabicyclo[3,2,1]oct-3-yl-méthyle, éventuellement substitué dans le cycle hétérocyclique à 6 chaînons par un ou deux groupes méthyle, et de leurs sels d'addition d'acides physiologiquement supportables avec des acides minéraux ou organiques, caractérisé en ce que

a) on acyle la pyridobenzodiazépinone de formule II

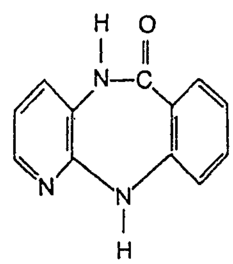

(II)

au moyen d'un dérivé d'acide de formule générale III

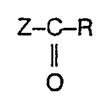

(III

dans laquelle

R est défini comme plus haut, et

Z représente un groupe nucléofuge, dans un solvant inerte à des températures entre $-25$ et $+130\,°C$ ou

b) pour la préparation de composés de formule générale I dans laquelle R représente un groupe (1-méthyl-1,2,5,6-tétrahydro-4-pyridinyl)méthyle ou 1-méthyl-1,2,5,6-tétrahydro-4-pyridinyle, éventuellement substitué dans le cycle hétérocyclique à 6 chaînons par un ou deux groupes méthyle, on réduit un sel de pyridinium de formule générale Va

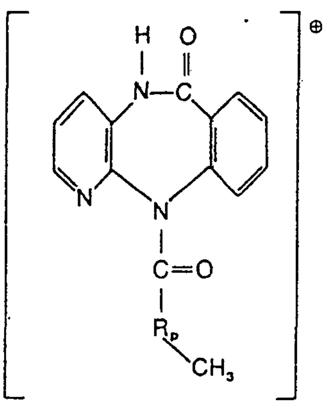

(Va)

dans laquelle

X représente le radical acide d'un acide oxygéné fort ou un atome d'halogène et

$R_P$ représente un groupe 4-pyridinyle ou (4-pyridinyl)méthyle, éventuellement substitué par un ou deux groupes méthyle, au moyen d'un borohydrure ou d'un alcoxyborohydrure dans des solvants protiques, à des températures entre −40 et +50 °C,

et éventuellement, on transforme ensuite un composé ainsi obtenu de formule générale I en ses sels physiologiquement supportables avec des acides minéraux ou organiques.

2. Procédé pour la préparation de nouvelles pyridobenzodiazépinones de formule générale I

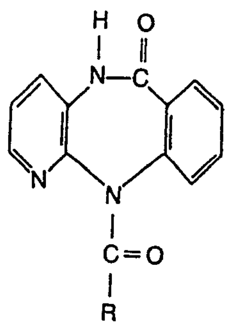

(I)

dans laquelle

R représente un groupe
(1-méthyl-4-pipéridinyl)méthyle,
(1-méthyl-1,2,5,6-tétrahydro-4-pyridinyl)méthyle,
1-méthyl-1,2,5,6-tétrahydro-4-pyridinyle,
(1-méthyl-4-pipéridinylidène)méthyle,
endo- ou
exo-8-méthyl-8-azabicyclo[3,2,1]oct-3-yl-méthyle,
éventuellement substitué dans le cycle hétérocyclique à 6 chaînons par un ou deux groupes méthyle, et de leurs sels d'addition d'acides physiologiquement supportables avec des acides minéraux ou organiques, caractérisé en ce que

a) on acyle la pyridobenzodiazépinone de formule II

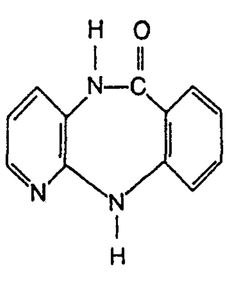

(II)

au moyen d'un dérivé d'acide de formule générale III

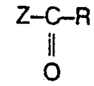

(III)

dans laquelle

R est défini comme plus haut et

Z représente un groupe nucléofuge, dans un solvant inerte, à des températures entre −25 et +130 °C ou

b) pour la préparation de composés de formule générale I dans laquelle R représente un groupe (1-méthyl-1,2,5,6-tétrahydro-4-pyridinyl)méthyle ou 1-méthyl-1,2,5,6-tétrahydro-4-pyridinyle, éventuellement substitué dans le cycle hétérocyclique à 6 chaînons par un ou deux groupes méthyle, on réduit un sel de pyridinium de formule générale Va

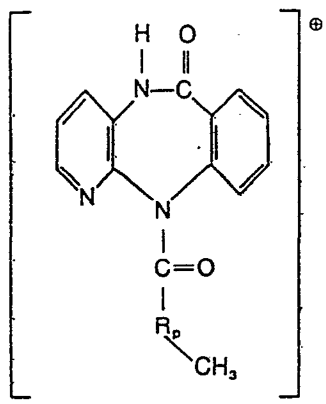

(Va)

dans laquelle

X représente le radical acide d'un acide oxygéné fort ou un atome d'halogène et

$R_P$ représente un groupe 4-pyridinyle ou (4-pyridinyl)méthyle, éventuellement substitué par un ou deux groupes méthyle,

au moyen d'un borohydrure ou d'un alcoxyborohydrure dans des solvants protiques à des températures entre −40 et +50 °C,

et éventuellement, on transforme ensuite un composé ainsi obtenu de formule générale I en ses sels physiologiquement supportables avec des acides minéraux ou organiques.

3. Procédé selon la revendication 1a), caractérisé en ce que l'on utilise, en tant que dérivé d'acide de formule générale III, un halogénure d'acide, un ester, un anhydride d'acide, un anhydride mixte d'acide ou un sel de N-alcoyl-2-acyloxypyridinium.

4. Procédé selon la revendication 1a) caractérisé en ce que l'on utilise, en tant que dérivé d'acide de formule générale III, l'anhydride mixte avec un acide minéral fort et en ce qu'on effectue la réaction dans un solvant inerte en présence d'un agent fixant les acides.

5. Procédé selon la revendication 1b), caractérisé en ce qu'on réduit un sel de pyridinium de formule générale Va au moyen de tétrahydroborate de sodium ou de potassium ou d'alcoxy-, dialcoxy- ou trialcoxyborohydrures de sodium ou

de potassium à −5 jusqu'à +10 °C en présence d'un solvant protique.

6. Procédé pour la préparation des composés de formule générale Va selon la revendication 1b), caractérisé en ce qu'on fait réagir la pyridobenzodiazépinone de formule II

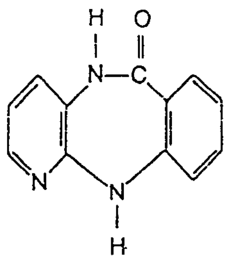

(II)

avec un agent d'acylation de formule générale IV

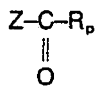

(IV)

dans laquelle

Z représente un groupe nucléofuge, et

$R_P$ représente un groupe 4-pyridinyle ou (4-pyridinyl) méthyle, éventuellement substitué par un ou deux groupes méthyle, dans un solvant inerte à des températures allant jusqu'à la température d'ébullition du mélange réactionnel, pour donner un composé de formule générale V

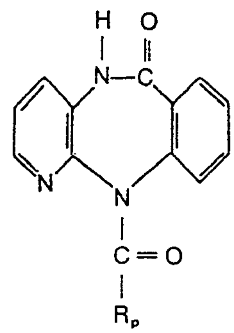

(V)

et en ce qu'ensuite on méthyle un composé ainsi obtenu au moyen d'un agent de méthylation de formule générale

$$H_3C-X \quad (VI)$$

dans laquelle

X représente le radical acide d'un acide oxygéné fort ou un atome d'halogène, dans un solvant inerte à des températures entre −20 et +130 °C.